(19) Europäisches Patentamt
European Patent Office
Office européen des brevets

(11) Publication number: **0 284 413**
**A2**

(12) **EUROPEAN PATENT APPLICATION**

(21) Application number: 88302669.2

(22) Date of filing: 25.03.88

(51) Int. Cl.4: **A 61 K 47/00**
C 12 N 11/06, C 07 D 213/34

(30) Priority: 27.03.87 GB 8707369

(43) Date of publication of application: 28.09.88 Bulletin 88/39

(84) Designated Contracting States: AT BE CH DE ES FR GB GR IT LI LU NL SE

(71) Applicant: BEECHAM GROUP PLC
Beecham House Great West Road
Brentford Middlesex TW8 9BD (GB)

(72) Inventor: Smith, Richard Anthony Godwin Beecham
Pharmaceuticals Great Burgh Yew Tree Bottom Road
Epsom Surrey KT18 5XQ (GB)

Kalindjian, Sarkis Barret Beecham Pharmaceuticals
Great Burgh Yew Tree Bottom Road
Epsom Surrey KT18 5XQ (GB)

(74) Representative: Valentine, Jill Barbara et al
Beecham Pharmaceuticals Patents & Trade Marks Dept.
Great Burgh Yew Tree Bottom Road
Epsom Surrey KT18 5XQ (GB)

The title of the invention has been amended (Guidelines for Examination in the EPO, A-III, 7.3).
Claims for the following Contracting State: ES.

(54) **Derivative of a fibrinolytic enzyme.**

(57) A derivative of a fibrinolytic enzyme in which the catalytic site on the enzyme which is responsible for fibrinolytic activity is blocked by a specific immunoglobulin or fragment thereof linked thereto by a reversible linking group and directed against an antigen associated with components of thrombotic material.

EP 0 284 413 A2

**Description**

## NOVEL COMPOUNDS

This invention relates to enzyme derivatives for use in the treatment and/or prophylaxis of thrombotic diseases.

European Patent No. 0,009,879 discloses derivatives of in vivo fibrinolytic enzymes which are useful therapeutic agents for treating venous thrombosis. The derivatives are characterised by the active catalytic site on the enzymes being blocked by a group which is removable by hydrolysis such that the pseudo first order rate constant for hydrolysis is in the range $10^{-6}$ to $10^{-3}$ $sec^{-1}$.

EP-A-0155388 discloses further derivatives of fibrinolytic enzymes in which the catalytic site on the enzyme which is responsible for fibrinolytic activity is blocked by a human protein attached thereto by way of a reversible linking group. Such proteins, when thus linked reversibly to the active sites of fibrinolytic enzymes can produce enzyme derivatives with slow physiological clearance rates.

It has now been found that certain proteins of the immunoglobulin class can, when coupled to the active sites of fibrinolytic enzymes using the reagents disclosed in EP-A-0155388, mediate the delivery of fibrinolytic activity to target sites defined by the binding specificities of the immunoglobulins.

According to the present invention, there is provided a derivative of a fibrinolytic enzyme in which the catalytic site on the enzyme which is responsible for fibrinolytic activity is blocked by a specific immunoglobulin or fragment thereof linked thereto by a reversible linking group and directed against an antigen associated with components of thrombotic material.

As used herein, the expression 'reversible linking group' includes groups which are removable by hydrolysis at a rate such that the pseudo first order rate constant for hydrolysis is in the range $10^{-6}$ $sec^{-1}$ to $10^{-3}$ $sec^{-1}$ in isotonic aqueous media at pH 7.4 at 37°C.

The preferred rate constant is in the range $1 \times 10^{-5}$ $sec^{-1}$ to $8 \times 10^{-4}$ $sec^{-1}$.

Suitably, the catalytic site on the enzyme is blocked by a group of structure I

(Im) -B-A-X (I)

in which

(Im) is a specific immunoglobulin as defined above, optionally modified by treatment with an amino acid side chain specific reagent to include a protein attachment group,

X is an acyl group of formula

$$-R-\overset{\overset{O}{\|}}{C}-$$

in which R is an aromatic or aliphatic residue,

A is a bridging group comprising at least one hetero atom selected from oxygen, sulphur and nitrogen, in which the nitrogen is optionally substituted by $C_{1-6}$ alkyl, and

B is a linear hydrophilic linking group, linked to the protein attachment group on (Im).

The term 'fibrinolytic enzyme' is used herein to mean any enzyme which demonstrates in vivo fibrinolytic activity as defined in European Patent No. 0009 879 (US 4285932). The term thus includes those enzymes which function by direct proteolysis of fibrin (such as plasmin) and those which function by activation of plasminogen (such as human tissue plasminogen activator [t-PA], urokinase (both high and low molecular weight and the single chain forms) and complexes of streptokinase with plasmin). Such enzymes are obtainable from mammalian urine, blood or tissues or by recombinant DNA methods such as where heterologous host organisms such as bacteria, yeasts, fungi or mammalian cell lines express genes specifying the enzymes. The term also includes:

(a) the fibrinolytically active hybrid proteins as disclosed in EP-A-0155387;

(b) the derivatives of fibrinolytic enzymes as disclosed in EP-A-0155388;

(c) the protein conjugates as disclosed in EP-A-0152736;

(d) the conjugates comprising a fibrinolytic enzyme linked to at least one water-soluble polymer by means of a reversible linking group as disclosed in EP-A-0183503;

(e) muteins of naturally occurring fibrinolytic enzymes such as those disclosed in EP-A-0201153 EP-0207589, WO-8604351 and EP-0199574.

As used herein, the term 'immunoglobulin' includes those serum and secretory proteins of classes IgG, IgA, IgD, IgM and IgE and the recognised subclasses thereof elicited by specific antigens in animal species including man, rabbits, sheep, goats, cows, horses, pigs and rodents. The term covers both the products of polyclonal immune response in animals and the products expressed by monoclonal hybridoma cells in culture. Also included are the fragments of immunoglobulin produced by proteolytic or chemical degradation of the intact proteins and which retain the domains responsible for specific antigen binding. Examples of such fragments include the monomeric and dimeric forms of the Fab domain. Immunoglobulins and their domains from heterologous expression of cDNA encoding the immunoglobulin heavy and light chains (or the fragments and hybrids thereof) are also included within the term.

The term 'specific' as used herein to describe immunoglobulins, refers to the property of high-affinity binding by such immunoglobulins to a single or restricted set of related chemical structures (antigenic

determinants) which characterise the thrombotic mass or the underlying pathological changes in blood vessel structure that may give rise either directly or indirectly to the thrombotic phenomena. Examples of such thrombus- or lesion-associated antigenic entities include the glycoprotein complexes IIb/IIIa and Ia of activated human platelets (Thrombos. Haemostas. 55, 153-157, 1986 and Blood 68, 783-786, 1986), antigenic determinants present in polymeric or monomeric fibrin (but not in the precursor fibrinogen) such as those exposed by the removal of fibrinopeptides A and B during clotting (Science 1983 vol. 222, 1129-1132), the membrane proteins of human erythrocytes (Biochem. Biophys. Acta. 1974 342, 69-80) and lymphocytes (Arthritis Rheum. 18, 1-8, 1975), collagen of the types found in atherosclerotic lesions and neoantigens expressed by cells of the vascular endothelium and subendothelium during development of atheromatous plaque (Clin. Sci. 69 supplement 12, 80p, 1985).

Suitable examples of specific immunolglobulins for use in this invention include rabbit polyclonal anti-(human erythrocyte membrane glycoprotein) immunoglobulin G (Biochem. Biophys. Acta. 1974 342, 69-80) and mouse monoclonal anti-(human fibrin) immunoglobulin G (Science 1983 vol. 222, 1129-1132).

Examples of suitable groups X include groups derived from those blocking groups described in European Patent No. 0,009,879. Preferred groups are optionally substituted benzoyl groups as described in the above mentioned European Patent further substituted at the 2 or 4 position by the group A, optionally substituted acryloyl groups also described in the above mentioned European patent and joined to A at the 2 or 3 position thereof and naphthoyl.

Suitable groups A are those which provide sufficient stabilisation of the resulting acyl-enzyme to result in a pseudo first order rate constant for hydrolysis in the above mentioned range and preferably in the range $1 \times 10^{-5}$ to $8 \times 10^{-4}$ $sec^{-1}$.

A preferably comprises one or two heteroatoms.

Examples of A are:

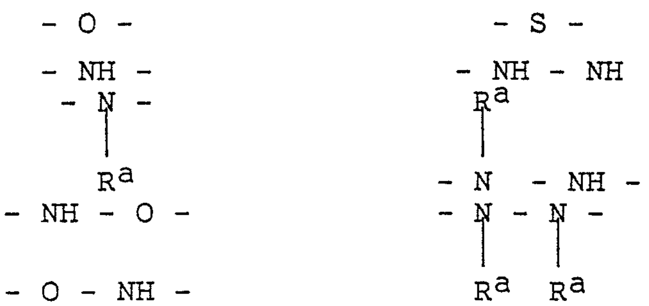

wherein $R^a$ is a $C_{1-6}$ alkyl group.

The protein attachment group is a functionality derived by modification of the immunoglobulin with a reagent specific for one or more amino acid side chains, and which contains a group capable of reacting with a group B.

Examples of groups B are substituted $C_2$-$C_{10}$ alkanes such as 6-aminohexyl, or linear polymers such as polyethylene glycol, polypropylene glycol, poly-glycine, poly-alanine or poly-sarcosine. The linear group B may optionally contain a cleavable section to facilitate analysis of the derivative or to react with the protein attachment group such as those derived from a 3-thio propionyl or 2-thio acetyl derivative of the -amino alkane or polymer function. An example of a cleavable section is a disulphide bond. Preferably the disulphide bond is derived from reaction of (Im) with the linear group B and thus is generated at the linkage of B with (Im). Alternatively the cleavable section may comprise an α, β dihydroxy function.

Preferably, B is a structure of the type $-S-(CH_2)_2-CONH-(CH_2)_n-$ where n is 0 to 8, more preferably 2 to 8. Examples of B include

$-S(CH_2)_2CONH-$, $-S-(CH_2)_2CONH(CH_2)_4-$,
$-S-(CH_2)_2CONH(CH_2)_3-$, $-S-(CH_2)_2CONH(CH_2)_6-$,
$-S-(CH_2)_2CONH(CH_2)_2-$, $-S-(CH_2)_2CONH(CH_2)_5-$ and
$-S-(CH_2)_2CONH(CH_2)_2O(CH_2)_2O(CH_2)_2-$.

As an example, the generation of a free thiol function by reaction of the specific immunoglobulin with 2-iminothiolane or N-acetyl homocysteine thiolactone will permit coupling of the protein attachment group with a thiol-reactive B structure. Alternatively, the protein attachment group can contain a thiol-reactive entity such as the 6-maleimidohexyl group or a 2-pyridyl-dithio group which can react with a free thiol in B. Preferably, the protein attachment group is derived from protein modifying agents such as 2-iminothiolane that react with lysine ε-amino groups in proteins.

Preferably, the protein (Im) in formula (I) will be of the IgG class, of mouse monoclonal origin and either modified to introduce 1-3 thiol groups or fragmented and partially reduced to give an Fab domain containing a single thiol function as described, for example, by L. Hudson and F.C. Hay 'Practical Immunology' 1, 196-199, Blackwell, Oxford, 2nd Ed. 1980.

The derivatives of the present invention may be prepared by reacting together a specific immunoglobulin optionally modified to include a protein attachment group, a fibrinolytic enzyme and a linking agent having a moiety capable of reacting with the catalytic site of the enzyme and a moiety capable of reacting with a protein amino group or protein attachment group to form a reversible linking group as hereinbefore defined.

In particular, the derivatives of the present invention may be prepared by reacting together a specific immunoglobulin which has been optionally modified by treatment with an amino acid side-chain specific reagent to include a protein attachment group, with an acylating agent of formula (II)

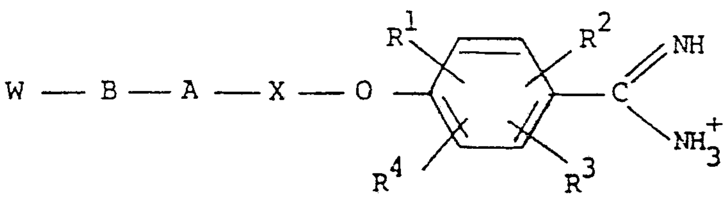

(II)

in which B, A and X are as defined with reference to formula (I), W represents a group capable of reacting directly with the amino acid side chain of a specific immunglobulin or, when the immunoglobulin includes a protein attachment group, W represents a group capable of reacting with the attachment group, Z represents a counter anion, preferably halide or p-toluenesulphonate, and each of $R^1$ to $R^4$ represents hydrogen or an electron withdrawing moiety which increases the reactivity of an amidinophenyl ester; and reacting the derivatised immunoglobulin produced thereby with a fibrinolytic enzyme.

Novel acylating agents of formula (II) form part of the invention.

In particular, the invention provides acylating agents of formula (III):

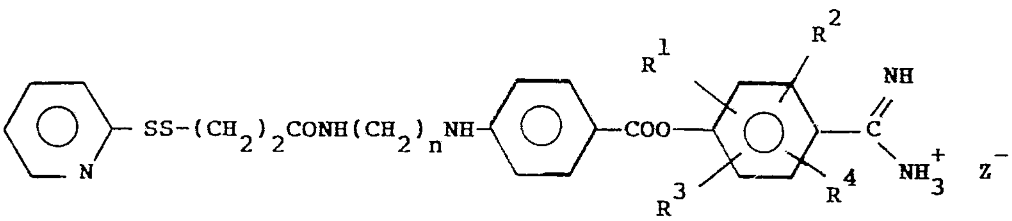

(III)

wherein n is an integer of 3 to 8, more preferably 3 to 6, Z is a counter ion and each of $R^1$ to $R^4$ represents hydrogen or a electron withdrawing moiety which increases the reactivity of an amidinophenyl ester.

It has been surprisingly found that when the length of the alkylene bridge in the compound of formula (III) is increased there is a corresponding reduction in the hydrolysis rate constant (k) of derivatives of the invention prepared from the compound.

The preferred range of rate constants for a derivative of the invention for use in the treatment of acute myocardial infarction is $1.9 \times 10^{-4}$ to $6.4 \times 10^{-5}$, corresponding to a deacylation half life of approximately 1-3 hours. The novel acylating agents of formula (III) allow derivatives of the invention to be prepared having rate constants within the preferred range.

When W represents a group capable of reacting directly with the amino acid side chain of a protein, it is preferably an N-succinimidyl group. When W represents a group capable of reacting with a protein attachment group, it is preferably a pyridylthio group.

Optionally W may be a photoactivated group such as 2-nitro-4-azido phenyl.

Preferably, each of $R^1$ to $R^4$ represents hydrogen or halogen.

The derivatives of the present invention may also be prepared by treating a specific immunoglobulin, which has been modified by treatment with an amino acid side chain specific reagent to include a protein attachment group, with a fibrinolytic enzyme which itself has been modified by treatment with an acylating agent of formula (II) in which W is a group capable of reacting with the protein attachment group on the protein.

Preferably, W is a pyridylthio group.

In the above processes, modification of the specific immunoglobulin to introduce a protein attachment group is preferably carried out in aqueous buffered media at a pH between 3.0 and 9.0 depending on the reagent used. For a preferred reagent, 2-iminothiolane, the pH is preferably 6.5-8.5. The concentration of immunoglobulin is preferably high (> 10mg/ml) and the modifying reagent is used in a moderate (1.1- to 5-fold) molar excess, depending on the reactivity of the reagent. The temperature and duration of reaction are preferably in the range 0°-40°C and 10 minutes to 7 days. The extent of modification of the protein may be determined by assaying the immunoglobulin for attachment groups introduced.

Such assays may be standard protein chemical techniques such as titration with 5,5′-dithiobis-(2-nitroben-

zoic acid). Preferably, 0.5-3.0 moles of protein attachment group will be introduced on average per mole of immunoglobulin. The modified immunoglobulin may be separated from excess modifying agents by standard techniques such as dialysis, ultrafiltration, gel filtration and solvent or salt precipitation. It is generally desirable to react the modified immunoglobulin with the acylating agent or the acylated fibrinolytic enzyme as soon as possible, but in certain cases, the intermediate material may be stored in frozen solution or lyophilised.

The modified immunoglobulin prepared as described above may be reacted with the acylating agent of formula (II) under conditions similar to those used for the initial introduction of the protein attachment group but with the following qualifications:

(a) To avoid hydrolysis of the amidinophenyl ester, the preferred pH range is 7.0 to 8.0 and non-nucleophilic buffers are preferred.

(b) The preferred temperature range is 0° C-30° C and the duration of reaction up to 6 hours.

(c) The molar ratio of acylating agent to protein attachment group is preferably in the range 1 to 10.

(d) The reaction may, optionally, be monitored by observing the release of a derivative of group W (e.g. pyridine 2-thione).

(e) Because of the reactivity of the product (a proteinaceous acylating agent), it is desirable to separate the product from the excess acylating agent of formula (II) as quickly as possible. For this purpose, high performance size exclusion chromatography or diafiltration may be used.

(f) The product should preferably be reacted with the fibrinolytic enzyme immediately but may be stored in frozen solution (not lyophilised) below -40° C for short periods.

The treatment of an unmodified specific immunoglobulin with an acylating agent of formula (II) is generally performed under conditions similar to those used for the introduction of a protein attachment group. However, the reactivity of this type of reagent requires that precautions similar to those noted in paragraphs (a) to (f) above should be exercised.

In the process aspect of the invention which utilises a reaction between modified specific immunoglobulin and acylated fibrinolytic enzyme, the enzyme may first be reacted with an acylating agent of formula (II) under the conditions described for the introduction of blocking groups in European Published Patent Application No. 0,009,879. Having been freed of excess reagent by the techniques noted above, the acylated enzyme may then be reacted with immunoglobulin containing a protein attachment group under conditions similar to those used in paragraphs (a) to (d) above. However, it is preferable to conduct the coupling below 10° C (preferably 0°-4° C) in order to minimise the hydrolysis of the acylated enzyme. In addition, the modified immunoglobulin may be used in a large molar excess over the acylated enzyme. The latter conditions also apply to the coupling between an a proteinaceous acylating agent and fibrinolytic enzyme.

The derivatives of the present invention may be separated from excess unreacted components by a variety of separation techniques. In general, the preferred techniques will exploit the binding specificities of both the fibrinolytic enzyme component and the immunoglobulin component. Thus, a conjugate of a specific immunoglobulin G with human tissue plasminogen activator may be purified:

1) by passage through a column of immobilised lysine to retain the plasminogen activator and remove immunoglobulin, or

2) by passage through columns containing either immobilised Staphylococcus Aureus Protein A or the antigen against which the immunoglobulin is directed (to remove excess plasminogen activator).

Alternatively and preferably, the latter step may be performed by gel permeation chromatography which exploits the difference in molecular weights of the conjugate and the unmodified plasminogen activator.

The acylating agents of formula (II) may be prepared by a variety of standard procedures. A preferred general synthetic route is given below.

(a) Reaction of an acylating agent containing a masked reactive functionality (for example, N-succinimidyl-3-(2-pyridyldithio)propionate-SPDP) with a 2- or 4- substituted benzoic acid derivative containing a nucleophilic function on the substituents (for example: 4-hydrazinobenzoic acid, N-2 (6-aminohexyl) aminobenzoic acid or N-4 (2-aminoethyl)aminobenzoic acid). Preferred reaction conditions require dry pyridine (or another basic solvent) at ambient temperature for 1-48 hours.

(b) The intermediate acid from (a) is esterified with a salt of 4-amidinophenol (or a ring-substituted derivative thereof) using dicyclohexylcarbodiimide in a weakly basic solvent as described for simple blocking agents in European Published Patent No. 0,009,879. It is preferable to use a slight molar excess of the amidinophenol (1.5 to 4-fold) to ensure efficient esterification. Optionally, the esterification may be performed in the presence of anhydrous p-toluenesulphonic acid as an acidic catalyst.

The invention also provides a process for preparing novel compounds of formula (III) which comprises esterifying an acid of formula (IV):

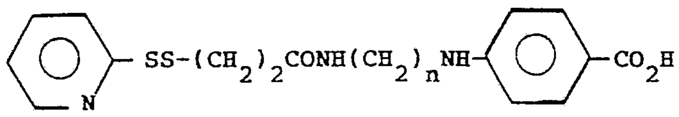

(IV)

with an alcohol of formula (V):

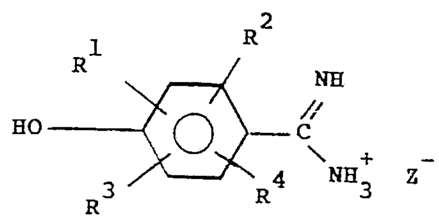

(V)

wherein the variables are as defined in formula (III).

Appropriately substituted benzoic derivatives for use in stage (a) may be prepared conventionally. For example, 4-fluorobenzoic acid may be esterified with a suitable alcohol such as t-butanol, and the fluoro group substituted by a suitable diamine at elevated temperature.

The derivatives of this invention are preferably administered as pharmaceutical compositions.

Accordingly, the present invention also provides a pharmaceutical composition comprising a derivative of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile derivative in sterile isotonic aqueous buffer. Where necessary the composition may also include a solubilising agent to keep the derivative in solution and a local anaesthetic such as lignocaine to ease pain at the site of injection. Generally, the derivative will be supplied in unit dosage form for example as a dry powder or water-free concentrate in a hermetically sealed container such as an ampoule or sachette indicating the quantity of derivative in activity units, as well as an indication of the time within which the free, unmodified protein will be liberated. Where the derivative is to be administered by infusion, the derivative will be dispensed with an infusion bottle containing sterile pharmaceutical 'water for injection'. Where the derivative is to be administered by injection the derivative is dispensed with an ampoule of sterile water for injection. The injectable or infusable composition will be made up by mixing the ingredients prior to administration.

The quantity of material administered will depend upon the amount of fibrinolysis required and the speed with which it is required, the seriousness of thromboembolic condition and position and size of the clot. The precise dose to be employed and mode of administration must per force in view of the nature of the complaint be decided according to the circumstances by the physician supervising treatment. However, in general, a patient being treated for a thrombus will generally receive a daily dose of from 0.10 to 10.0 mg/kg of body weight either by injection in up to five doses or by infusion. For the treatment of coronary thrombosis a similar dose may be given as a single intravenous bolus.

The derivatives of this invention may also be used for the prevention of thrombotic disorders by administration of similar doses to the patient at a time prior to the anticipated thrombotic challenge. For example, prevention or amelioration of deep venous thrombosis consequent upon the trauma of hip replacement surgery may be achieved by the administration of an agent capable of releasing fibrinolytic activity at a potential thrombus site over a period of hours after the generation of that site by surgical trauma. Such a derivative would be administered immediately before (or possibly during) the operative procedure.

No adverse toxicological effects are indicated with the compounds of the invention within the above described dosage range.

Accordingly, in a further aspect of the invention there is provided a method of treatment and/or prophylaxis of thrombotic disorders, which comprises administering to the mammal in need of such treatment and/or prophylaxis an effective non-toxic amount of a derivative of the invention.

The invention also provides the use of a derivative of the invention for the preparation of a medicament for the treatment and/or prophylaxis of thrombotic disorders.

The following Methods and Examples illustrate the invention.

## Methods

(a) Chromogenic substrate assay

Urokinase and t-PA were assayed against the chromogenic substrates (KabiVitrum, Sweden) S-2444 and S-2288, respectively at a substrate concentration of 1 mM in 0.1 M triethanolamine.HCl pH 8.0 at 25°C. An SU

is defined as the amount of activity that gives an O.D. increase of 0.001/min in 1 ml substrate in a 1 cm pathlength cell.

(b) Rate constant determinations

The pseudo first order rate constant is determined by hydrolysing the acyl-enzyme under physiological conditions, i.e. in isotonic aqueous media at pH 7.4 and at 37° C. At regular intervals aliquots are withdrawn and incubated with a chromogenic substrate and the rate of conversion of the substrate measured as indicated above.

The hydrolysis is followed until such time as the rate of conversion of substrate reaches a maximum. The rate constant k may then be calculated by plotting:

$Log_e\ (1\text{-}A_t/A_{max})$ against t

where $A_{max}$ is the maximum rate at which an aliquot converts substrate and $A_t$ is the rate at which an aliquot converts substrate at time t.

Preferably such rate constants are calculated by computerised non-linear regression analysis, fitting the $A_t$ and time data to the equation:

$A_t = A_o + (A_{max}\text{-}\ A_o)\ (1\text{-}e^{-kt})$

where $A_o$ is the activity of the acyl-enzyme preparation before deacylation.

(c) Fibrin plate assay of fibrinolytic activity

Fibrin plates were prepared from 0.4% (w/v) human fibrinogen (Kabi, Grade 1, Flow Laboratories, Scotland) dissolved in 0.029 M barbitone in 125 mM NaCl, pH 7.4, pipetted (9ml) into 10 × 10 cm square plastic dishes (Sterilin) and clotted by rapid mixing with 0.3 ml bovine thrombin (50 NIH units/ml, Parke-Davis, UK). Plates were incubated at 37° for 18-24h usually, but longer if required, and stained with aqueous bromophenol blue. For each lysis zone, two diameters perpendicular to each other were measured using Vernier calipers. All diameters for each sample were averaged, and this mean converted to fibrinolytic activity by reference to a calibration curve.

Example 1

Active centre-linked conjugate of human high molecular weight urokinase with rabbit polyclonal anti-(human erythrocyte membrane glycoprotein) immunoglobulin G prepared using 4′-amidinophenyl 4-N-[2-N-(3-[2-pyridyldithio]propionyl)aminoethyl]aminobenzoate

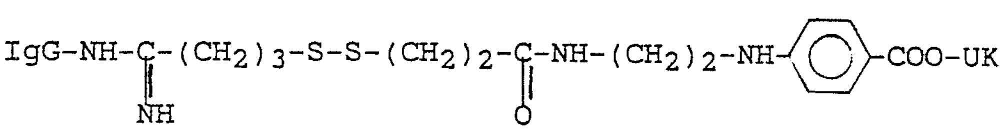

(a) N-ε-(4-thiobutyrimino)-[LYSINE] rabbit anti-(human erythrocyte membrane glycoprotein) immunoglobulin G

Rabbit polyclonal IgG raised against human erythrocyte glycoproteins as described by O.J. Bjerrum and P. Lundahl (Biochem. Biophys. Acta. 1974 342, 69-80), [3.0 ml of 30 mg/ml in 0.90/w/v sodium chloride] was mixed with 0.05 M sodium phosphate, 0.1 M sodium chloride, 0.01% w/v Tween 80 detergent pH 7.4 [phosphate buffer] (1.0 ml) and freshly prepared 2-iminothiolane (50 µl of 50 mM in water) added. The mixture was incubated for 30 min at 25° C and then precipitated by addition of saturated ammonium sulphate solution (4.0 ml) and centrifuging for 20 min at 9000g/4° C. The pellet was dissolved in 0.5 M L-arginine, 0.5 M sodium chloride, 20 mm Trishydroxymethylaminomethane.hydrochloride, 0.01% w/v Tween 80 detergent pH 7.4 (ANT buffer, 2.0 ml) and gel filtered on a small column (PD10) of Sephadex G-25 into ANT buffer (3.0 ml) at 4° C. The solution contained approximately 25 mg/ml of protein and titration of free thiol with 5.5 dithiobis(2-nitrobenzoic acid) indicated a sulphydryl content of 136 µM, i.e. an average level of substitution of 0.84 mol thiol/mol protein. The product was used at once.

(b) 4-[2-N-(3-[2-pyridyldithio]propionyl)aminoethyl] aminobenzoyl human high molecular weight urokinase

High MW urokinase ($10^6$ IU, approx. 105 nmol) was dissolved in phosphate buffer (1.0 ml) and 4′-amidinophenyl 4-N-[2-N-(3-[2-pyridyldithio] propionyl)aminoethyl]aminobenzoate (example 3f of EP-A-0155388, 25 µl of 20 mM in dry dimethylsulphoxide solution) added. The mixture was incubated 30 min at 25° C and the addition and incubation repeated. The amidolytic activity of the urokinase declined to 2.4% of its initial value. The product was freed of excess acylating agent by gel filtration at 4° C on a small column (PD10) of Sephadex G-25 into ANT buffer (3.0 ml) and used immediately.

(c) Coupling and purification of the title compound 4-N-[2-N′-(3-[4′-butyrimino-(N″-ε-lys rabbit anti-[human erythrocyte membrane glycoprotein] immunoglobulin G)]dithiopropionyl)aminoethyl] aminobenzoyl-O-(ser-356) human high molecular weight urokinase.

The above solutions were mixed and bovine lung trypsin inhibitor (Aprotinin, 0.5 ml of 2.9 mg/ml) added. The volume of the mixture was reduced by centrifugal concentration (Amicon Centricon-10, at 2°C, 5000g for 2.5h) to about 2.0 ml and the product was stored at -196°C. The whole sample was applied to a 300 × 21.5 mm column of TSK G3000 SWG HPLC gel permeation matrix equilibrated and eluted with ANT buffer at 20-23°C, 2.0 ml/min. Protein peaks were detected eluting at around 72 ml, 86 ml and 118 ml. Material eluting between 68 and 88 ml was pooled, gel filtered (PD10, as above) into 0.2% w/v D-mannitol, 20 mM ammonium bicarbonate, 1.0 mM 6-aminohexanoic acid, pH 7.4 [21 ml], lyophilised in 15 × 1.4 ml lots, and stored at -40°C. The product deacylated at 37°C in phosphate buffer with an average rate constant of about 2.9 × $10^{-4}$ $sec^{-1}$.

Example 2

Active centre-linked conjugate of human tissue plasminogen activator with rabbit polyclonal anti-(human erythrocyte membrane glycoprotein) immunoglobulin G prepared using 4′-amidinophenyl 4-N-[4-N-(3-[2-pyridyldithio]propionyl) aminoethyl]aminobenzoate

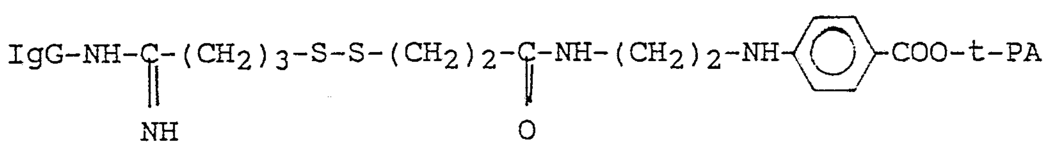

(a) N-ε-(4-thiobutyrimino)-[LYSINE] rabbit anti-(human erythrocyte membrane glycoprotein) immunoglobulin G

The rabbit antibody of Example 1 (63 µl of 30 mg/ml, 12 nmol) was added to 0.9% w/v sodium chloride (0.9 ml) and buffered with 1.0 M sodium phosphate, 0.01% w/v Tween 80 detergent pH 7.4 (0.1 ml). A freshly prepared solution of 2-iminothiolane (25 µl of 100 mM in water) was added and the mixture incubated at 25°C for 30 min. Saturated ammonium sulphate solution (2.0 ml) was added and the product isolated by centrifugation at 5000g for 40 min at 4°C. The pellet was dissolved in the phosphate buffer of Example 1 (0.5 ml) and the precipitation step repeated. The final solution (0.5 ml) was titrated with 5,5 dithiobis-(2-nitrobenzoic acid) and contained 79 µM free thiol groups, corresponding to about 3.2 mol thiol/mol protein. The product was used immediately.

(b) 4-[2-N-(3-[2-pyridyldithio]propionyl)aminoethyl] aminobenzoyl human tissue plasminogen activator

Purified t-PA (approx. 100 nmol in the ANT buffer of Example 1, 2.0 ml) was mixed with 4′-amidinophenyl 4-N-[4-N-(3-[2-pyridyldithio]propionyl)aminoethyl] aminobenzoate (50µl of 20mM in dry dimethylsulphoxide) and incubated for 20 min at 25°C. After this time, the amidolytic activity of the enzyme was reduced by about 95%. The product was gel filtered using a small (PD10) column of Sephadex G-25 at 4°C into ANT buffer (3.4 ml). Reduction of an aliquot of this solution with 2.5 mM dithiothreitol and monitoring of released pyridine 2-thione at 343 nM indicated that the product contained about 102 nmol of the 2-pyridyldithio function. The material was stored at -196°C.

(c) Coupling and purification of title compound 4-N-[2-N′-(3-[4′-butyrimino-(N″-ε-lys rabbit anti-[human erythrocyte membrane glycoprotein] immunoglobulin G)]dithiopropionyl)aminoethyl] aminobenzoyl-O-(ser-478) human tissue plasminogen activator.

The whole of the above thiolated IgG product was mixed with part of the product from (b) above (1.5 ml, 45 nmol) and the volume reduced by centrifugal concentration (conditions as in Example 1 but over 2h only) to about 0.2 ml). The product was chromatographed as described in Example 1. A pool of fractions (2.0 ml) eluting between 64 and 72 ml was retained, stored temporarily at -70°C and then concentrated by centrifugal concentration to about 1.0 ml. The final product was stored at -196°C. The product deacylated at 37°C in phosphate buffer with an average first order rate constant of 1.92 × $10^{-4}$ $sec^{-1}$.

Example 3

Active centre-linked conjugate of human tissue plasminogen activator with rabbit polyclonal anti-(human erythrocyte membrane glycoprotein) immunoglobulin G prepared using 4′-amidinophenyl 4-N-[8-N-(3-[2-pyridyldithio]propionyl)amino-3,6-dioxaoctyl] aminobenzoate

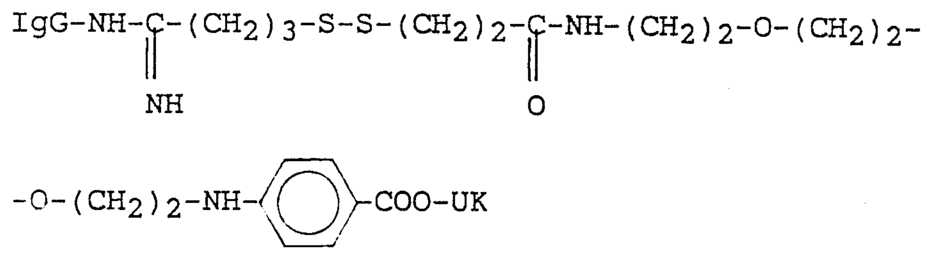

(a) t-Butyl 4-fluorobenzoate

t-Butanol (6.6 ml) and pyridine (5.62 ml) were dissolved in dry dichloromethane (40 ml) and cooled in a dry ice/acetone bath to -50°C. A solution of 4-fluorobenzoic acid chloride (10.0 g) in dry dichloromethane (20 ml) was added slowly to the alcohol solution. The solution was kept under nitrogen throughout, and allowed to warm slowly to room temperature. It was then heated at reflux for 1d. More t-butanol (1 ml) and pyridine (1 ml) were added and reflux continued for two days more. After cooling the reaction mixture was washed successively with 2 M hydrochloric acid (40 ml), 10% sodium hydrogen carbonate solution (40 ml) and saturated aqueous sodium sulphate solution. The organic layer was dried, filtered and evaporated to leave an oil (10.62 g). This contained the title compound and some 4-fluorobenzoic anhydride. The two components were separated by column chromatography (100 g silica/ 30% dichloromethane/70% petroleum ether) to leave the title compound (8.48 g, 69%) $^1H$ NMR ($CDCl_3$) δ 8.05, 7.05 (4H, m, aryl-H) and 1.60 (9H, S, $CH_3$). Infra Red (Thin Film) $V_{max}$ 2970, 1715, 1610, 1510, 1290, 1155, 1120, 850, and 770 $cm^{-1}$.

(b) t-Butyl 4-N-(8-amino-3,6-dioxaoctyl)aminobenzoate

t-Butyl 4-fluorobenzoate from (a) above (1.0 g) was dissolved in 1,8-diamino-3,6-dioxaoctane (4 ml) and heated to 120°C under an atmosphere of dry nitrogen for 3d. Water (5 ml) was added and the solution was basified with 5 M sodium hydroxide solution. The aqueous layer was extracted with ethyl acetate (2 × 10 ml). The organic layer was re-extracted with saturated sodium sulphate solution (5 ml) and then dried filtered and evaporated to leave an oil (1.42 g, 86%) the title compound.

$^1H$ nmr ($CDCl_3$) δ 7.8 (2H,d,J:8Hz, aryl-H), 6.55 (2H,d,J:8Hz, aryl-H), 4.9 (1H, brs, aryl-NH), 3.5 (10H,m, 4 × $CH_2O$ + $CH_2NH$ aryl), 2.8 (2H,t,J=5Hz, $CH_2NH_2$), and 1.55 (11H,S,$CCH_3$ + $NH_2$). Infra red (Thin Film), $V_{max}$3370, 1690, 1610, 1530, 1290, 1160, 1110, and 770$cm^{-1}$.

A di-p-toluenesulphonic acid salt mp 117-8°C was produced. Found C,54.79; H,6.58; N,3.92. $C_{17}H_{28}N_2O_4 \cdot 2 C_7H_8SO_3 . ^1/_2H_2O$ requires C,54.93; H,6.69; N,4.13%.

(c) t-Butyl 4-N-[8-N-(3-[2-pyridyldithio]propionyl) amino-3,6-dioxaoctyl]aminobenzoate

t-Butyl 4-N-(8-amino-3,6-dioxaoctyl)aminobenzoate from (b) above (110 mg) was dissolved in pyridine (1 ml) and N-succinimidyl-3-(2-pyridyldithio)propionate (SPDP)(106 mg) was added. The solution was stirred at room temperature, under nitrogen, for 3d. After this time, pyridine was removed by evaporation and ethyl acetate (5 ml) was added and the organic layer was extracted with 10% aqueous sodium hydrogen carbonate solution (5 ml). The organic layer was dried, filtered and evaporated to leave a yellow oil (127 mg). This was purified by chromatography (1 g silica/ethyl acetate) to leave the title compound (90 mg, 51%).

$^1H$ nmr ($CDCl_3$) δ 8.5(1H,m,2-pyridyl-H), 7.75 (4H,m,2× aryl-H + 2× pyridyl-H), 7.2 (1H,m,pyridyl-H), 6.6 (3H,m,aryl-H + NHCO), 4.6 (1H,s,aryl-N-H), 3.65 (14H,m, 2× NCH + 4× $OCH_2$ + $COCH_2$), 2.6(2H,t,J=5Hz,$CH_2S$), and 1.55(9H,s,$CCH_3$). Infra Red (Thin Film) $V_{max}$ 3350, 1690, 1670, 1610, 1290 and 1160 $cm^{-1}$.

Mass Spectrum FAB in 3-nitrobenzyl alcohol (3-NOBA) shows 522 ($MH^+$), 466, 410, 337, 305, 289, 219, 206.

(d) 4-N-[8-N-(3-[2-pyridyldithio]propionyl)amino-3,6-dioxaoctyl]aminobenzoic acid ditrifluoroacetate salt

The t-butyl ester prepared in (c) above (90 mg) was suspended in trifluoroacetic acid (2 ml) and left to stand at room temperature overnight. The solution was filtered through a plug of glass wool and the volatile material was removed by evaporation. This left the title compound (120 mg, 100%) a pale yellow oil.

$^1H$ nmr $d^6$acetone / $CDCl_3$ δ 8.7(1H,d,J=4Hz,2-pyridyl-H), 7.9)5H,m,aryl-H + NHCO), 6.8 (2H,d,J=8Hz, aryl-H), 3.6(14H,m,2× $NCH_2$ + 4× $OCH_2$ + $COCH_2$), and 2.7(2H,t,J=6Hz, $CH_2S$).

(e) 4'-Amidinophenyl 4-N-[8-N-(3-[2-pyridyldithio] propionyl)amino-3,6-dioxaoctyl]diaminobenzoate Hydrochloride Salt

The acid-trifluoracetate salt from (d) above (212 mg) was dissolved in pyridine (1 ml). 4-Amidinophenol (53 mg) was added followed by dicyclohexylcarbodiimide (DCCI) (64 mg). The reaction was stirred under an atmosphere of dry nitrogen for 4d. The solution was then filtered, evaporated and the orange gum triturated with chloroform (2×3 ml). The gum was then dissolved in ethanol (0.5 ml) and filtered. Diethyl ether was added

until the product, a gum precipitated. This was isolated by decantation and evaporation to leave the title compound (11 mg) a pale brown gum. 1H nmr showed this material to be about 60% pure, the major impurity being 4-amidinophenol hydrochloride.

$^1$H nmr d$^6$DMSO δ 9.25 (4H,brd,$D_2O$ exchangeable, $^+NH_2$), 8.75(1H,S,NH), 8.45(1H,m,2-pyridyl-H), 7.8(6H,m,2× aryl-H + 2× amidinophenyl-H + 2× pyridyl-H), 7.5(2H,d,J=9Hz, amidinophenyl-H), 7.25(1H,m,pyridyl-H), 6.80(1H,t, 2Hz,aryl-NH), 6.70(2H,d,J=9Hz,aryl-H), 3.60(14H,m, 4× $OCH_2$ + 2× $NHCH_2$ + $COCH_2$) and 3.0(2H,t,J=8Hz, $CH_2S$-S).

FAB mass spectrum in 3-NOBA:584 ($M^+$ for free cation).

(f) 4-N-[8-N-(3-[2-pyridyldithiolpropionyl)amino-3,6-dioxaoctyl]aminobenzoyl human tissue plasminogen activator

Purified t-PA (approx. 77 nmol in ANT buffer, 1.6 ml) was mixed with the acylating agent from (e) above (20 µl of 83 mM in dry dimethylsulphoxide) and held at 0°C for 16h. After this time, the amidolytic activity of the enzyme was reduced by more than 98%. The product was gel filtered on a small (PD10) column of Sephadex G-25 at 4°C into ANT buffer (3.5 ml) and stored at -196°C.

(g) Coupling to thiolated IgG and purification of title compound 4-N-[8-N′-(3-[4′-butyrimino-(N″-ε-lys rabbit anti-[human erythrocyte membrane glycoprotein]immunoglobulin G)]dithiopropionyl) amino-3,6-dioxaoctyl]aminobenzoyl-O-(ser-478) human tissue plasminogen activator.

Thiolated rabbit anti-(human erythrocyte membrane glycoprotein) IgG was prepared as in Example 1(a) except that 30 µl of the 2-iminothiolane solution was used. The product contained about 0.76 mol thiol/mol protein. This material was mixed with the product from (f) above and then concentrated to a volume of approx. 2.5 ml and chromatographed as described in Example 1(c) above. Assay of individual fractions by partial hydrolysis of aliquots at 37°C and amidolytic determination of t-PA using substrate S-2288, indicated that the peak of high-molecular weight activity eluted at about 69 ml (low molecular weight activity - i.e. uncoupled acyl-t-PA - eluted at about 90 ml). A pool of fractions eluting between 62 and 76 ml was precipitated with saturated ammonium sulphate solution (16 ml) at 10,000g, 4°C for 30 min. The reconstituted pellet was gel filtered as described above into phosphate buffer (3.5 ml) and stored at -196°C. The product deacylated at 37°C in phosphate buffer with an average first order deacylation rate constant of about 2.5 × $10^{-4}$ $sec^{-1}$.

Example 4

Active centre-linked conjugate of human tissue plasminogen activator with rabbit polyclonal anti-(human erythrocyte membrane glycoprotein) immunoglobulin G prepared using 4′-amidinophenyl 4-N-[4-N-(3-[2-pyridyldithio]propionyl)aminobutyl]aminobenzoate

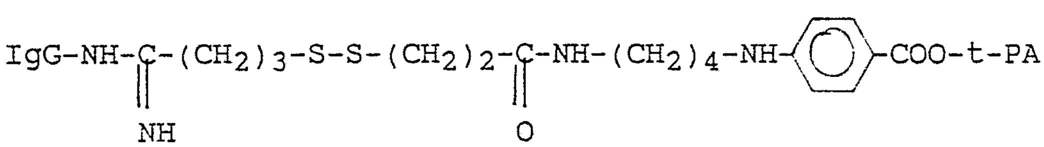

(a) t-Butyl 4-N-(4-aminobutyl)aminobenzoate

t-Butyl 4-fluorobenzoate (1.0 g) was mixed with 1,4-diaminobutane (10 ml) and the mixture was heated to 120°C overnight. After cooling, water (10 ml) was added followed by 5 M sodium hydroxide solution (4 ml). The aqueous solution was extracted with ethyl acetate (2 × 15 ml), and the organic layer was dried (sodium sulphate), filtered and evaporated. This left the title compound, contaminated with the starting diamine. The mixture was taken up in ethyl acetate (50 ml) and washed with saturated sodium sulphate solution (20 ml). The organic layer was dried, filtered and evaporated to leave the title compound (1.02 g, 76%).

$^1$H nmr ($CDCl_3$) δ 7.8 (2H,d,J=8Hz), 6.55(2H,d,J=8Hz), 4.5 (1H,br s), 3.1(2H,t,J=7Hz), 2.7(2H,t,J=7Hz) and 1.55(15H,br s).

$V_{max}$ 3370, 3225, 1690, 1600, 1530, 1290, 1160 and 1115 $cm^{-1}$

A 4-toluenesulphonic acid salt was prepared and recrystallised from ethanol and ether m.p. 134-7°. Found C,59.68; H,7.57; N,6.54%. $C_{22}H_{32}N_2SO_5$ 1/4$H_2O$ requires C,59.90; H,7.42; N,6.54%.

(b) t-Butyl 4-N-[4-N-(3-[2-pyridyldithio]propionyl) aminobutyl]aminobenzoate

t-Butyl 4-N-(4-aminobutyl)aminobenzoate (169 mg) from (a) above was dissolved in pyridine (2 ml). SPDP (200 mg) was added and the reaction stirred at room temperature under an atmosphere of dry nitrogen for 2d. The pyridine was removed by evaporation and the oil was taken up in ethyl acetate (5 ml). The organic layer was washed with 10% sodium hydrogen carbonate solution (2 ml). The organic layer was dried, filtered and evaporated to yield a solid (219 mg). The title compound was isolated by chromatography (2 g silica/ethyl

acetate) as a solid (166 mg, 56%) m.p. 98-9° from dichloromethane and petroleum ether.
$^1H$ NMR ($CDCl_3$) 8.5(1H,m), 7.8 (4H,m), 7.1 (1H,m), 6.9 (1H,m), 6.6 (2H,d,J=8Hz), 4.2(1H,s), 3.2(6H,m), 2.6(2H,t,J=6Hz) and 1.55 (13H,m).
Infra red $V_{max}$ 3350, 1680, 1660, 1605, 1530, 1300, 1160, 1120, 910, 835, and 730 $cm^{-1}$.
Found C,58.95; H,6.74; N,9.04. $C_{23}H_{31}N_3O_3S_2$. $^1/_2H_2O$ requires C,58.69; H,6.85; N,8.92%.

(c) 4-N-[4-N-(3-[2-Pyridyldithio]propionyl) aminobutyl]aminobenzoic acid

The t-butyl ester prepared in (b) above (153 mg) was dissolved in trifluoroacetic acid (2 ml) and allowed to stand at room temperature for 24h. The solution was filtered and evaporated to leave a yellow oil (290 mg) which was a trifluoroacetate salt of the title compound. The material was used without further purification.
$^1H$ nmr ($d^6$ acetone/$CDCl_3$) δ 8.3(1H,m), 8.1(5H,m), 3.3(6H,m), 2.8(2H,t,J=5Hz), and 1.8(4H,brs).

(d) 4′-Amidinophenyl 4-N-[4-N-(3-[2-pyridyldithio] propionyl)aminobutyl]aminobenzoate

The acid-trifluoroacetate salt from (c) above (216 mg) was dissolved in pyridine (1 ml) and 4- amidinophenol (45 mg) was added. DCCI (54 mg) was also introduced. The solution was stirred for 3d under an atmosphere of nitrogen at room temperature. The solution was filtered and evaporated. The oil was taken up in chloroform (200 µl) and precipitated with petroleum ether. The oil was separated by decantation and was successively triturated with chloroform (2 ml), water (2 ml), brine (2 ml) and finally water (2 ml). After drying in vacuo, the oil was dissolved in ethanol (2 ml), filtered and evaporated. The oil was taken up in a small quantity of ethanol (200 µl) and precipitated with diethyl ether. After decantation this final procedure was repeated and the product (40 mg) dried in vacuo. This material was about 40% the title compound with large amounts of the two products of hydrolysis present (the starting acid and the phenol). Repeat precipitation of the product led to material that was over 80% pure.
$^1H$ nmr ($d^6$DMSO) δ 9.25(4H,brd), 8.35(1H,m), 7.80(6H,m), 7.50(2H,d,J=9Hz), 7.25(1H,m), 6.80(1H,t,J=5Hz), 6.65(2H,d,J=9Hz), 3.1(8H,m), 1.5(4H,m).
FAB Mass Spectrum in 3-Nitrobenzylalcohol $M^+$ of free base at m/e 524, 460, 440, 423.

(e) N-ε-(4-thiobutyrimino)-[LYSINE] rabbit anti-(human erythrocyte membrane glycoprotein) immunoglobulin G

The rabbit antibody of Example 1 (0.5 ml of 30 mg/ml in 0.9% w/v sodium chloride) was diluted to 2.0 ml in phosphate buffer and treated with a fresh solution of 2-iminothiolane (30 µl of 100 mM in water) for 30 min at 25°C. The product was gel filtered into phosphate buffer (3.4 ml) as detailed above. Thiol titration, as above, indicated a free sulphydryl content of about 69 µM and an average substitution of 2.4 mol thiol/mol protein. The product was used immediately.

(f) 4-N-[4-N-(3-[2-pyridyldithio]propionyl)amino butyl]aminobenzoyl human tissue plasminogen activator

Purified t-PA (2.0 ml in ANT buffer, 112 nmol) was treated with the acylating agent from (d) above in solution in dry dimethylsulphoxide in two stages: 1) 25 µl of 10 mM for 30 min at 25°C, 2) 25 µl of 50 mM for 2.5h at 25°C. After this time, 98.6% of the initial amidolytic activity was lost. The product was gel filtered into ANT buffer (3.4 ml) as detailed above and reduction (as in Example 2(b)) indicated a pyridyldithio group content of 37 µM. The material was stored at -196°C.

(g) Coupling and purification of title compound 4-N-[2-N′-(3-[4′-butyrimino-(N″-ε-lys rabbit anti-[human erythrocyte membrane glycoprotein] immunoglobulin G)]dithiopropionyl)amino butyl]aminobenzoyl-O-(ser-478) human tissue plasminogen activator.

The whole of the thiolated IgG from (e) above was mixed with the product from (f) (1.0 ml, 37 nmol) and the volume of the mixture reduced by centrifugal concentration (as in Example 1(c) but for 1.5h) to 0.5 ml. The product was diluted to 1.5 ml with ANT buffer and gel filtered (as above) into phosphate buffer (3.4 ml). The solution was applied to a column (0.8 × 2.0 cm) of L-lysine- Sepharose (Pharmacia) and washed with phosphate buffer (5.0 ml). The column was eluted with ANT buffer (5.0 ml) and the eluate concentrated (as above) to about 1.0 ml. The product was chromatographed as described in Example 1(c) and fractions eluting between 66 and 74 ml pooled and concentrated (as above) to about 1.2 ml. The final product was stored at -196°C.
The average first order deacylation rate constant in phosphate buffer at 37°C was $1.65 \times 10^{-4}$ $sec^{-1}$.

Example 5

Active centre-linked conjugate of human tissue plasminogen activator with a mouse monoclonal anti-(human fibrin) immunoglobulin G prepared using 4′-amidinophenyl N-4-[N-2-(3-[2-pyridyldithio] propionyl)aminoethyl]aminobenzoate

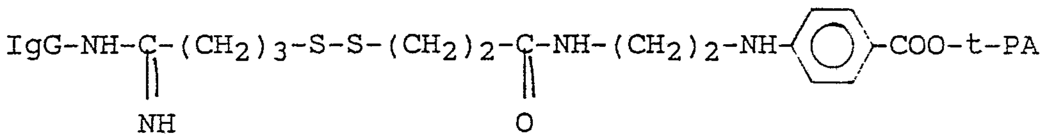

(a) N-ε-(4-thiobutyrimino)-[LYSINE] mouse anti-(human fibrin) immunoglobulin G

Purified mouse monoclonal antibody directed against an antigen generated upon conversion of human fibrinogen to fibrin (Code: Y22, W. Nieuwenhuizen et al., Fibrinolysis (8th International Congress on Fibrinolysis) Abstract No. 139; 1.7 mg in 0.9 ml 0.9% w/v sodium chloride) was mixed with 1.0 M sodium phosphate buffer, 0.01% Tween 80 pH 7.4 (0.1 ml). The buffered solution was treated twice with a fresh solution of 2-iminothiolane (25 μl of 100 mM) (firstly for 30 min and secondly for 15 min, in both cases at 25° C) and then gel filtered as described above into the phosphate buffer of Example 1(a) (3.4 ml). The product was used immediately.

(b) Coupling to 4-[N-2-(3-[2-pyridyldithio]propionyl) aminoethyl]aminobenzoyl human tissue plasminogen activator and purification of title compound 4-N-[2-N'-(3-[4'-butyrimino-(N" = ε-lys mouse monoclonal anti-[human fibrin]immunoglobulin G)]dithiopropionyl)amino ethyl]aminobenzoyl-O-(ser-478) human tissue plasminogen activator.

The product of Example 2(b) (1.5 ml, 45 nmol) was added to the whole of the above sample and the mixture concentrated by centrifugal concentration to a small volume (conditions as in Example 2(c)), diluted to about 1.5 ml with ANT buffer and reconcentrated to about 0.5 ml over 1.5h. The product was chromatographed as described in Example 1(c) and fractions eluting between 64 and 76 ml pooled and reconcentrated (4000g, 40° C 1.75h) to a final volume of about 1.5 ml. The material was stored at -196° C.

The average first order deacylation rate constant in phosphate buffer at 37° C was $2.9 \times 10^{-4}$ $sec^{-1}$.

## Example 6

Active centre-linked conjugate of human tissue plasminogen activator with rabbit polyclonal anti-(human erythrocyte membrane glycoprotein) immunoglobulin G prepared using 4'-amidinophenyl 4-N-[6-N-(3-[2-pyridyldithio]propionyl)aminohexyl] aminobenzoate

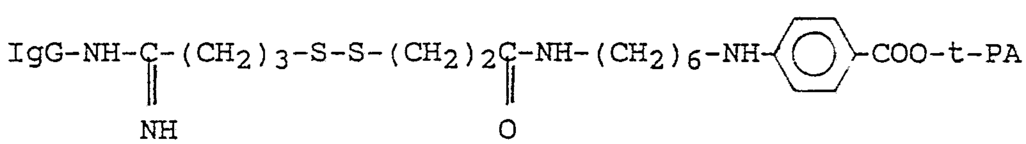

(a) t-Butyl 4-N-(6-aminohexyl)aminobenzoate

t-Butyl 4-fluorobenzoate (0.5g) was dissolved in 1,6-diaminohexane (5ml). The mixture was heated to 120° C under an atmosphere of dry nitrogen for 3d. Water (5ml) was added, followed by 5M sodium hydroxide solution (2ml). The aqueous layer was extracted with ethyl acetate (10ml), and the organic layer was washed successively with 2M sodium hydroxide solution (10ml) and water (10ml). The ethyl acetate layer was dried, filtered and evaporated to leave a yellow solid (517mg, 69%) which was the title compound.

$^1$H nmr ($CDCl_3$) δ, 7.8(2H,d, J = 9Hz, aryl-H), 6.55(2H,d,J = 9Hz, aryl-H), 4.1(1H, br s, aryl-N-H), 3.15(2H, br t, aryl-NHC$H_2$), 2.7(2H,t, J = 6Hz, $CH_2NH_2$), 1.6(9H,s,C$CH_3$), 1.5(8H,m, 4 × C$H_2$), νmax 3370, 1690, 1610, 1530, 1480, 1370, 1295, 1160, 1120, 910, and 735$cm^{-1}$.

(b) t-Butyl 4-N-[6-(3-[2-pyridyldithio]propionyl) aminohexyl]aminobenzoate

The amine from (a) (187mg) was dissolved in pyridine (2ml) and SPDP (200mg) was added. The reaction was stirred under an atmosphere of dry nitrogen for 3d. The solution was evaporated and the residue taken up in ethyl acetate (2ml). This was washed with 10% aqueous sodium hydrogen carbonate solution (2ml). The organic layer was dried, filtered and evaporated to leave a solid (212mg). This was chromatographed (2g, silica/ethyl acetate) to leave the title compound (131mg, 42%), m.p. 104-5°.

$^1$H nmr ($CDCl_3$)δ, 8.5 (1H,m,aryl-H), 7.9(4H,m,aryl-H), 7.2(1H,m,pyridyl-H), 6.6(3H,d,aryl-H + N-H CO), 4.0(1H,br s, N-H aryl), 3.1(6H,m,$CH_2$-N + $CH_2$CO), 2.6(2H,t, J = 6Hz, $CH_2$), and 1.6(17H,M, $CH_3$ + 4 × $CH_2$),

vmax 3350, 1680, 1650, 1605, 1530, 1415, 1295, 1160, 1115, 830, and 760$cm^{-1}$.
Found: C,61.67; H,7.28; N,8.54. $C_{25}$ $H_{35}$ $N_3$ $S_2$ $O_3$ requires: C,61.32; H,7.20; N 8.53%.

(c) 4-N-[6-N-(3-[2-pyridyldithio]propionyl)aminohexyl] aminobenzoic acid

The t-butyl ester from (b) (113mg) was dissolved in trifluoroacetic acid (2ml). The solution was left to stand overnight and then filtered and evaporated to leave an orange oil (159mg). This was the ditrifluoroacetate salt of the title compound.

$^1$H nmr $d^6$ acetone/$CDCl_3$ δ 8.7(1H,m, aryl-H), 8.1(6H,m, 5 × aryl-H + NHCO), 7.2(2H,d,J=9Hz, aryl-H), 4.4(1H,br s, aryl-NH), 3.3(6H,m, $CH_2N$ + $CH_2CO$), 2.8(2H,t,J=4Hz, $CH_2S$), 1.4(8H,m, 4×$CH_2$).

(d) 4′-Amidinophenyl 4-N-[6-N-(3-[2-pyridyldithio] propionyl)aminohexyl]aminobenzoate Hydrochloride salt.

The acid ditrifluoroacetate salt from (c) (159mg) and 4-amidinophenol (42mg) were dissolved in dry pyridine (1ml) and DCCI (100mg) was added. The solution was stirred at room temperature under an atmosphere of dry nitrogen for 7d, and in this time two more aliquots of DCCI (100mg each) were added. After this time, the solution was filtered and evaporated. The residual orange gum was successively triturated with chloroform (2 × 2ml), water (2ml), brine (2ml) and finally water (2ml). The residual gum was dried by evaporation and ethanol (1ml) was added. The solution was filtered and diethyl ether was introduced. The title compound precipitated as a foam (30mg). This was 75% pure by $^1$H nmr. The only significant contaminant was the starting acid.

$^1$H nmr ($d^6$DMSO) δ 9.22 (4H,s, amidine N-H), 8.45(1H,m,pyridyl-H), 7.8(6H,m, 2 × aryl-H + 2 × pyridyl-H), 7.50 (2H,d,J=9Hz, phenol-H), 7.25 (1H,m,pyridyl-H), 6.8(1H,t,J:4Hz, aryl-NH), 3.3(2H,m, $CH_2NH$), 3.0(6H,m, $CH_2CO$ + $CH_2NH$ Ar + ($CH_2S$), and 1.3 (8H,m, 4×$CH_2$).

(e) N-[ε-(4-thiobutyrimino)]-[lysine]rabbit anti-(human erythrocyte membrane glycoprotein) immunoglobulin G

The rabbit antibody of Example 1 (0.2ml of 30mg/ml in 0.9% w/v sodium chloride) was diluted to 2.2ml in phosphate buffer and treated with a fresh solution of 2-iminothiolane (20µl of 100 mm in phosphate buffer) for 30 minutes at 25°C. The product was gel filtered into phosphate buffer as detailed above. Thiol titration, as above, indicated a free sulphydryl content of 33.5µm and an average substitution of 2.9 mol. thiol/mol protein. The product was used immediately.

(f) 4-N-[6-N-(3-[2-pyridyldithio]prooionyl)aminohexyl] aminobenzoyl human tissue plasminogen activities

Purified t-PA (1.0 ml in ANT buffer, 54nmol) was treated with the acylating agent from (d) above (50 µl of 10mm in dry dimethylsulphoxide) for 1h at 25°C. After this time, 96% of the initial amidolytic activity was lost. The product was gel-filtered into ANT buffer (3.4ml) and stored at -196°C.

(g) Coupling and Purification of title compound 4-N-[2-N′-(3-[4′-butyrimino-(N″-ε-lys rabbit anti-[human erythrocyte membrane glycoprotein] immunoglobulin G)]dithiopropionyl)aminohexyl]aminobenzoyl-O-(ser-478) human tissue plasminogen activator.

The whole of the thiolated 1gG from (e) above was mixed with the whole of acyl-enzyme from (f), held 16h at -40°C and then concentrated (as described in Example 4(g)) to a volume of 1.5ml. The product was gel filtered into phosphate buffer (3.4ml) as described above and applied to a column of lysine-sepharose as detailed in Example 4(g). The column was washed with phosphate buffer (10ml) and the product was eluted with ANT buffer (10ml). The eluate was treated with saturated ammonium sulphate solution (10ml) and the product precipitated at 10,000g for 30 min at 4°C. The pellet was dissolved in ANT buffer (0.5ml and applied to a 600 × 7.5mm column of TSK G3000 SWG HPLC gel permeation matrix (with guard column) equilibrated and eluted with ANT buffer at 20-23°C, 0.5 ml/min. Fractions of 0.3 ml were collected and protein peaks eluting at 13.9 and 18.9 ml were detected. A pool of fractions eluting between 13.3 and 14.8ml was made and stored at -196°C. The product deacylated at 37°C in phosphate buffer with an average rate constant of 9.99 × $10^{-5}$ $sec^{-1}$.

Example 7

Active centre-linked conjugate of human tissue plasminogen activator with rabbit polyclonal anti-(human erythrocyte membrane glycoprotein)immunoglobulin G prepared using 4′-amidinophenyl 4-N-(3-N-[3-(2-pyridyldithio)propionyl]aminopropyl)aminobenzoate.

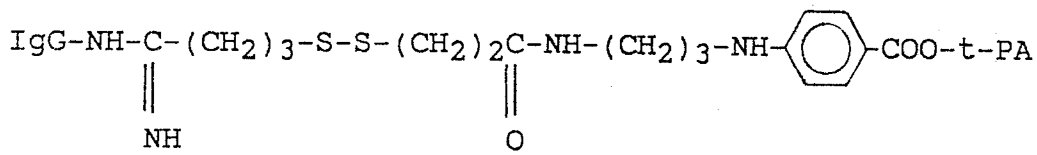

(a) t-butyl 4-N-(3-aminopropyl)aminobenzoate

t-Butyl 4-fluorobenzoate (0.5g) was dissolved in 1,3-diaminopropane (2.5ml) and the reaction heated to 120°C under an atmosphere of dry nitrogen for 16h, after which time no starting material could be detected by t.l.c. The solution was allowed to evaporate at reduced pressure and water (10ml) was added, followed by enough concentrated hydrochloric acid to solubilise the material. The aqueous layer was extracted with dichloromethane (10ml), which was discarded. The aqueous layer was basified with aqueous sodium hydroxide solution and then extracted with ethyl acetate (2 × 10ml). The organic layer was dried, filtered and evaporated to leave the title compound (488mg, 77%).

$^1$H nmr. ($CDCl_3$) δ, 7.82 (2H,d,J=9Hz, aryl-H), 6.55 (2H,d,J=9Hz, aryl-H), 4.7 (1H, br s, aryl-N-H),3.30 (2H,t,J=7Hz, $NHCH_2$-), 2.85 (2H,t,J=7Hz, $CH_2NH_2$), 1.8 (2H,m,$CH_2CH_2CH_2$), 1.6 (9H,s,$CCH_3$), and 1.3 (2H, brs, $NH_2$). νmax (Nujol), 3370, 1685, 1610, 1535, 1290, 1160, 920, 840, and 770 $cm^{-1}$, m/e found 250.1695, $C_{14}H_{22}N_2O_2$ requires 250.1681.

(b) t-Butyl 4-N-[3-N-(3-[2-pyridyldithio]propionyl) aminopropyl]aminobenzoate

The amine from (a) (100mg) and SPDP (125mg) were dissolved in pyridine (1ml). The reaction was stirred at room temperature under an atmosphere of dry nitrogen for 72h. The pyridine was removed by evaporation and ethyl acetate (5ml) was added. The organic layer was washed with 10% sodium hydrogen carbonate solution (2ml). This was then dried, filtered and evaporated to leave a gum (142mg) which was almost pure. Final purification was achieved by column chromatography (2g silica, ethyl acetate) to leave the title compound (95mg, 53%).

$^1$H nmr ($CDCl_3$) δ, 8.45 (1H,m,6-pyridyl-H), 7.7 (4H,m,aryl-H), 7.1 (2H,m,aryl-H+NHCO), 6.55 (2H,d,J=9Hz, aryl-H), 4.5 (1H, br s, aryl-NH), 3.3 (6H,m,$NHCH_2$ × 2 + $COCH_2$), 2.6 (2H,t, J=6Hz, $CH_2S$), 1.8(2H,t,J=6Hz, $CH_2CH_2CH_2$) and 1.55 (9H, s,$CCH_3$). νmax (Nujol) 3350, 1680, 1660, 1610, 1530, 1470, 1330, 1160, 1120, 910, 835, and 730 $cm^{-1}$.

(c) 4-N-[3-N-(3-[2-pyridyldithio]propionyl) aminopropyl]aminobenzoic acid

The t-butyl ester from (b) (85mg) was dissolved in trifluoroacetic acid, and allowed to stand at room temperature overnight. The volatile material was removed by evaporation to leave an oil (117mg). This was found to be the di-trifluoroacetate salt of the title compound and was used without further purification.

$^1$H nmr ($D^6$ acetone/$CDCl_3$) δ, 8.8 )1H,d, pyridyl-H), 8.0 (9H,m, 2 × aryl-H + pyridyl-H), 6.9 (2H,d, J =8Hz, aryl-H), 3.3 (6H,m 2 × $NCH_2$), 2.8 (2H, t,J=6Hz, $CH_2S$) and 1.9 (2H,m, $CH_2CH_2CH_2$).

(d) 4′-Amidinophenyl 4-N-(3-N-[3-(2-pyridyldithio) propionyl]aminopropyl)aminobenzoate hydrochloride salt.

The acid di-trifluoroacetate from (c) (117mg) was dissolved in pyridine (1ml), and 4-amidinophenol (3.4mg) was added followed by DCCI (41 mg). The solution was stirred under an atmosphere of dry nitrogen for 18h. The pyridine was removed by evaporation and the residue triturated successively with chloroform (1ml), water (1ml), brine (1ml) and finally water (1ml). The solution was dried under vacuum and ethanol (0.5ml) was added to the brown residue. The solution was filtered and diethylether (5ml) was added. The solution went cloudy and a brown oil was precipitated. After decantation, the oil was dried in vacuo and became a foam (20mg), the title compound, which by $^1$H nmr was about 80% pure.

$^1$H nmr ($D^6$DMSO) δ 9.29 (4H,s,amidine-NH), 8.45 (1H,d,J=4Hz aryl-H), 8.06 (1H,m CONH), 7.80 (6H,m, 2 × aryl-H + 2 × amidinophenyl-H + 2 × pyridyl-H), 7.48 (2H,d,J=8Hz, aryl-H from amidinophenol), 7.25 (1H,m, aryl-H pyridine), 6.66 (2H,d, J=8Hz, aryl-H); 5.59 (1H,d, J= 8Hz, aryl -NH), 3.3 (4H,m, 2 × $NHCH_2$), 3.15 (2H,t, J=6Hz, $COCH_2$), 3.05 (2H,t, J=6Hz,$CH_2SS$), and 1.25 (2H,m, $CH_2CH_2CH_2$).

(e) 4-N-[3-N-(3-(2-pyridylthio]propionyl)amino propyl]aminobenzoyl human tissue plasminogen activator.

Purified two-chain t-PA (2.0 ml in ANT buffer, 108 nmoles) was treated with the acylating agent from (d) above (50 µl of 20 mM solution in dry dimethylsulphoxide for 30 min at 25°C. The product was held on ice for 30 min and gel filtered into ANT buffer (3.4 ml). The product was precipitated by addition of saturated ammonium sulphate solution (7.6 ml), centrifuged for 30 min at 10,000 g/4°C and the precipitate dissolved in 0.9 ml ANT buffer. The product contained approximately 92 nmoles of pyridyldithio function as determined by reduction (example 2 (b)) and was stored at -196°C.

(f) Thiolation, coupling and purification of title compound 4-N-[2-N′-(3-[4′-butyrimino-(N″-ε-lys rabbit anti-[human erythrocyte membrane glycoprotein]immunoglobulin G)]dithiopropionyl) aminopropyl]-aminobenzoyl-O-(ser-478) human tissue plasminogen activator.

The rabbit antibody of example 1 (1.0 ml of a 13.3 mg/ml solution in 0.9% sodium chloride) was diluted to 2.5 ml in phosphate buffer, adjusted to pH 7.4 and treated with 2-iminothiolane (20µl of 100 nm in water) for 30 min at 25°C. It was then gel filtered into phosphate buffer as described above and found to contain 63 µm free sulphydryl groups (2.5 mol thiol/mol protein). The whole product was mixed with the acyl-t-PA from (e) above (0.9 ml) and the volume reduced by centrifugal concentration as described in example 4 (g) above to a volume of 1.5 ml. The product was gel filtered into phosphate buffer (3.4 ml) as described above and applied to a column (0.8 × 3.0 cm) of lysine-Sepharose (Pharmacia), washing with phosphate buffer (13.6 ml). The product was eluted with arginine buffer (9.0 ml), and precipitated with saturated $(NH_4)_2S_4$ solution (9.0 ml, 10,000 g/30 min/2°C). The pellet was dissolved in ANT buffer (0.4 ml) and applied to a column (7.5 mn × 60 cm) of TSK

G3000 SWG HPLC gel and eluted with ANT buffer at 0.5 ml/min, 20-23° C. The protein peak eluting between 13.0 and 14.8 ml was collected and held at -196° C. This product deacylated in phosphate buffer at 37° C with an average first order deacylation rate constant of 1.79 × $10^{-4}$ $sec^{-1}$.

## Example 8

### 4'-Amidinophenyl 4-N-[5-N-(3-[2-pyridyldithio] propionyl)aminopentyl]aminobenzoate Hydrochloride salt

(a) t-Butyl 4-N-(5-aminopentyl)aminobenzoate

t-Butyl 4-fluorobenzoate (0.5 g) was dissolved in 1,5-diaminopentane (5 ml) and the reaction heated to 100C under an atmosphere of dry nitrogen. The reaction was left for 48h after which time no starting material could be detected by tlc. Water (5ml) was added and the aqueous layer was basified with 5M aqueous sodium hydroxide solution and then extracted with ethyl acetate (10 ml). The organic layer was dried, filtered and evaporated to leave the title compound contaminated with the starting diamine. The material was taken up in ethyl acetate (10 ml) and washed successively with 5M sodium hydroxide (10 ml) and water (10 ml). The organic layer was dried, filtered and evaporated to leave the product (0.51 g, 72%). 'H n.m.r. ($CDCl_3$) δ 7.8 (2H, d, J=9Hz), 6.4 (2H, d, J=9Hz), 4.1 (1H, br s) 3.1 (2H, m), 2.7 (2H, t, J=5Hz), 1.5 (17H, m).

(b) t-Butyl 4-N-[5-N-(3-[2-pyridyldithio]propionyl) aminopentyl]aminobenzoate

The amine (178 mg) and SPDP (200 mg) were dissolved in pyridine (2 ml). The reaction was stirred under an atmosphere of dry nitrogen for 48h. The pyridine was removed by evaporation and ethyl acetate (5 ml) was added. The organic layer was washed with 10% aqueous sodium hydrogen carbonate solution (1 ml). The organic solution was dried, filtered and evaporated to leave a gum which was almost pure. However, the final purification was achieved by column chromatography (silica, ethyl acetate) to leave the product (140 mg, 46%).
'H n.m.r. ($CDCl_3$) δ 8.4 (1H, m), 7.7 (4H, m), 7.1 (1H, m), 6.8 (1H, t, J=5Hz), 6.45 (2H, d, J=8Hz), 4.35 (1H, t, J=6Hz), 3.1 (6H, m), 2.6 (2H, t, J=6Hz), 1.55 (15H, s).

(c) 4-N-[5-N-(3-[2-pyridyldithio]propionyl) aminopentyl]aminobenzoic acid

The t-butyl ester (130 mg) prepared above was dissolved in trifluoroacetic acid (1 ml) and left at room temperature for 48h. The solution was filtered and evaporated to leave an oil (192 mg). This was the acid in the form of a trifluoroacetate salt. The material was used for further transformations without any more purification.
'H n.m.r. $d^6$acetone/$CDCl_3$) δ 8.7 (1H, m), 7.9 (7H, m), 6.9 (2H, d, J=8Hz), 3.2 (6H, m), 2.7 (2H, t, J=5Hz), 1.5 (6H, br s).

(d) Title Ester

The acid (192 mg solid, 115 mg actual acid) prepared above was dissolved in pyridine (1 ml) and 4-amidinophenol (47 mg) was added followed by DCCI (113 mg). The solution was stirred under an atmosphere of dry nitrogen for 7d. The solution was filtered to remove the precipitated dicyclohexylurea and the pyridine was removed by evaporation. The residue triturated successively with chloroform (3 × 1 ml), water (1 ml), brine (1 ml) and finally water (1 ml). The solution was dried in vacuo and ethanol (3 ml) was added to the brown residue. The solution was filtered and concentrated. Diethyl ether (5 ml) was added and a brown solid was precipitated. The solution was decanted away and solid dried. This proved to be the title compound (31 mg, 19%), which was about 80% pure by proton n.m.r.
'H n.m.r. ($d^6$DMSO) δ 9.35 (4H, br s), 8.45 (1H, m), 7.8 (7H, m), 7.5 (2H, d, J=9Hz), 7.25 (1H, m), 6.80 (1H, t, J=5Hz,) 6.65 (2H, d, J=9Hz), 3.1 (6H, m), 1.55 and 1.40 (6H, m). 2 protons are not visible. These have probably been masked by the DMSO peak at 2.5 ppm.

## Demonstration of specific binding to human erythrocytes bv the compound of Example 1

(a) Method

Freshly collected whole citrated human blood (0.6 ml) was mixed with one aliquot of the lyophilised preparation of Example 1 dissolved in 0.05 M sodium phosphate, 0.1 M sodium chloride, 10 mg/ml human serum albumin pH 7.4 (PBSA buffer, 0.2 ml) and agitated gently for 10 min at 4° C. As controls, a comparable lyophilised preparation of a conjugate prepared in the same way as Example 1 but using human non-specific IgG (NSI control), a sample of the compound of Example 1 hydrolysed in phosphate buffer at 37° C for 200 min (Hydrolysis control), normal saline or unmodified urokinase were used in the procedure. The samples were diluted with PBSA buffer (3.0 ml), shaken briefly and centrifuged at 2000g, 4° C for 2 min. The supernatent was removed and the pelleted erythrocytes washed five times with PBSA buffer (3.5 ml), centrifuging as before. The final cell preparation was suspended in PBSA buffer (0.75 ml, total volume approx. 1.0 ml) and shaken gently in a water bath at 37° C. At various intervals up to 180 min, aliquots (100 µl) were removed and added to PBSA (100 µl) in microtubes held on ice. The samples were centrifuged as above and the tops cut off the microtubes to facilitate removal of the supernatent. The latter was stored at -40° C until assay. Urokinase assay was performed either with substrate S-2444 or by fibrinolytic assay on calibrated human fibrin plates (see 'Methods' section).

(b) Results

Figure 1 shows that a release of urokinase-like amidolytic activity could be detected from erythrocytes exposed to the compound of Example 1 but not from cells exposed either to pre-hydrolysed compound or to unmodified urokinase.

Figure 2 shows that the released activity was fibrinolytic and also that neither the conjugate with non-specific IgG nor untreated erythrocytes themselves could release such activity.

In the figures:

Figure 1Release of urokinase amidolytic activity into buffer medium containing washed human erythrocytes exposed to the compound of example 1 (●),
pre-hydrolysed compound (□)
and unmodified urokinase (▲)

Figure 2Release of urokinase fibrinolytic activity into buffer medium containing washed human erythrocytes exposed to the compound of Example 1 (●) or
NSI/saline controls (both □)

Both FiguresMeans ± s.e.m. (n = 4)
x-axis: minutes at 37°C
y-axis: urokinase released into medium (nM)

**Claims**

1. A derivative of a fibrinolytic enzyme in which the catalytic site on the enzyme which is responsible for fibrinolytic activity is blocked by a specific immunoglobulin or fragment thereof linked thereto by a reversible linking group and directed against an antigen associated with components of thrombotic material.

2. A derivative according to claim 1 in which the catalytic site on the enzyme is blocked by a group of structure I

(Im) -B-A-X (I)

in which

(Im) is a specific immunoglobulin as defined above, optionally modified by treatment with an amino acid side chain specific reagent to include a protein attachment group,

X is an acyl group of formula

$$-R-\overset{O}{\overset{\|}{C}}-$$

in which R is an aromatic or aliphatic residue,

A is a bridging group comprising at least one hetero atom selected from oxygen, sulphur and nitrogen, in which the nitrogen is optionally substituted by $C_{1-6}$ alkyl, andB is a linear hydrophilic linking group, linked to the protein attachment group on (Im).

3. A derivative according to claim 2 in which -B-A-X-has the structure

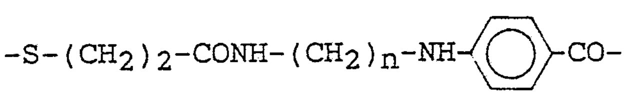

where n is an integer of 2 to 8.

4. A derivative according to any preceding claim wherein the linking group is derived from:

4′-amidinophenyl 4-N-[4-N-(3-[2-pyridyldithio] propionyl)aminobutyl]aminobenzoate;

4′-amidinophenyl 4-N-[6-N-(3-[2-pyridyldithio] propionyl)aminohexyl]aminobenzoate;

4′-amidinophenyl 4-N-(3-N-[3-(2-pyridyldithio) propionyl]aminopropyl)aminobenzoate;

4-amidinophenyl 4′-N-[5-N-(3-[2-pyridyldithio] propionyl)aminopentyl]aminobenzoate;

4′-amidinophenyl 4-N-[8-N-(3-[2-pyridyldithio] propionyl)amino-3,6-dioxaoctyl]aminobenzoate; or

4′-amidinophenyl 4-N-[2-N-(3-[2-pyridyldithio] propionyl)aminoethyl]aminobenzoate.

5. A derivative according to any preceding claim wherein the fibrinolytic enzyme is a plasminogen activator.

6. A derivative according to claim 5 wherein the fibrinolytic enzyme is tissue plasminogen activator or high molecular weight urokinase.

7. A derivative according to any preceding claim wherein the specific immunoglobulin is rabbit anti-[human erythrocyte membrane glycoprotein] immunoglobulin G or mouse monoclonal anti-(human fibrin) immunoglobulin G.

8. 4-N-[2-N′-(3-[4′-Butyrimino-(N″-ε-lys rabbit anti-[human erythrocyte membrane glycoprotein] immunoglobulin G)]dithiopropionyl) aminoethyl] aminobenzoyl-O-(ser-356) human high molecular weight urokinase,

4-N-[2-N′-(3-[4′-butyrimino-(N″-ε- lys rabbit anti-[human erythrocyte membrane glycoprotein] immunoglobulin G)]dithiopropionyl)aminoethyl] aminobenzoyl-O-(ser-478) human tissue plasminogen activator,

4-N-[8-N′-(3-[4′-butyrimino-(N″-ε- lys rabbit anti-[human erythrocyte membrane glycoprotein] immunoglobulin G)]dithiopropionyl)amino-3,6-dioxaoctyl] aminobenzoyl-O-(ser-478) human tissue plasminogen activator,

4-N-[2-N′-(3-[4′-butyrimino-(N″-ε-lys rabbit anti-[human erythrocyte membrane glycoprotein] immunoglobulin G)]dithiopropionyl)aminobutyl] aminobenzoyl- O-(ser-478) human tissue plasminogen activator,

4-N-[2-N′-(3-[4′-butyrimino-(N″-ε-lys rabbit anti-[human erythrocyte membrane glycoprotein] immunoglobulin G)]dithiopropionyl)amino butyl]aminobenzoyl- O-(ser-478) human tissue plasminogen activator,

4-N-[2-N′-(3-[4′-butyrimino-(N″-ε-lys rabbit anti-[human erythrocyte membrane glycoprotein] immunoglobulin G)]dithiopropionyl)aminohexyl] aminobenzoyl-O-(ser-478) human tissue plasminogen activator or

4-N-[2-N′-(3-[4′-butyrimino-(N″-ε-lys rabbit anti-[human erythrocyte membrane glycoprotein] immunoglobulin G)]dithiopropionyl)aminopropyl]aminobenzoyl-O-(ser-478) human tissue plasminogen activator.

9. A process for preparing a derivative according to claim 1 which process comprises reacting together a specific immunoglobulin optionally modified to include a protein attachment group, a fibrinolytic enzyme and a linking agent having a moiety capable of reacting with the catalytic site of the enzyme and a moiety capable of reacting with a protein amino group or protein attachment group to form a reversible linking group as hereinbefore defined.

10. A pharmaceutical composition comprising a derivative according to claim 1 in combination with a pharmaceutically acceptable carrier.

11. A derivative according to claim 1 for use as an active therapeutic substance.

12. A derivative according to claim 1 for the treatment and/or prophylaxis of thrombotic disorders.

13. The use of a derivative of claim 1, for the preparation of a medicament for the treatment and/or prophylaxis of thrombotic disorders.

14. A compound of formula (III)

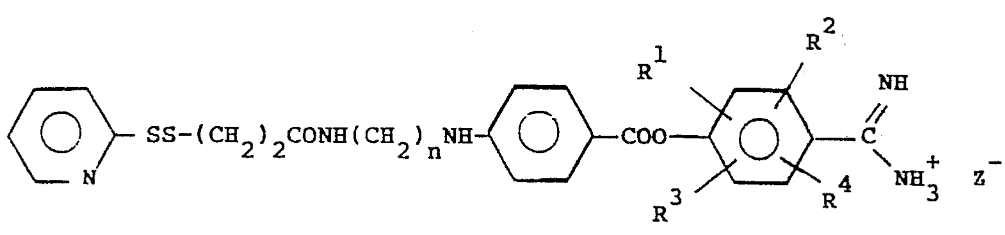

(III)

wherein n is an integer of 3 to 8, Z is a counter ion and each of $R^1$ to $R^4$ represents hydrogen or an electron withdrawing moiety which increases the reactivity of an amidinophenyl ester.

15. 4′-Amidinophenyl 4-N-[4-N-(3-[2-pyridyldithio] propionyl)aminobutyl]aminobenzoate,

4′-amidinophenyl 4-N-[6-N-(3-[2-pyridyldithio] propionyl)aminohexyl]aminobenzoate,

4′-amidinophenyl 4-N-(3-N-[3-(2-pyridyldithio) propionyl]aminopropyl)aminobenzoate or

4′-amidinophenyl 4′-N-[5-N-(3-[2-pyridyldithio] propionyl)aminopentyl]aminobenzoate, or a salt thereof.

Claims for the following Contracting State : ES

1. A process for preparing a derivative of a fibrinolytic enzyme in which the catalytic site on the enzyme which is responsible for fibrinolytic activity is blocked by a specific immunoglobulin or fragment thereof linked thereto by a reversible linking group and directed against an antigen associated with components of thrombotic material, which process comprises reacting together a specific immunoglobulin optionally modified to include a protein attachment group, a fibrinolytic enzyme and a linking agent having a moiety capable of reacting with the catalytic site of the enzyme and a moiety capable of reacting with a protein amino group or protein attachment group to form a reversible linking group as hereinbefore defined.

2. A process according to claim 1 in which the catalytic site on the enzyme is blocked by a group of structure I

(Im) -B-A-X (I)

in which

(Im) is a specific immunoglobulin as defined above, optionally modified by treatment with an amino acid side chain specific reagent to include a protein attachment group,

X is an acyl group of formula

$$-R-\overset{O}{\overset{\|}{C}}-$$

in which R is an aromatic or aliphatic residue,

A is a bridging group comprising at least one hetero atom selected from oxygen, sulphur and nitrogen, in which the nitrogen is optionally substituted by $C_{1-6}$ alkyl, andB is a linear hydrophilic linking group, linked to the protein attachment group on (Im).

3. A process according to claim 2 in which -B-A-X-has the structure

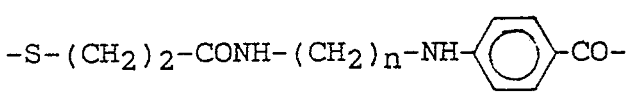

where n is an integer of 2 to 8.

4. A process according to any preceding claim wherein the linking group is derived from:

4′-amidinophenyl 4-N-[4-N-(3-[2-pyridyldithio] propionyl)aminobutyl]aminobenzoate;

4′-amidinophenyl 4-N-[6-N-(3-[2-pyridyldithio] propionyl)aminohexyl]aminobenzoate;

4′-amidinophenyl 4-N-(3-N-[3-(2-pyridyldithio) propionyl]aminopropyl)aminobenzoate;

4-amidinophenyl 4′-N-[5-N-(3-[2-pyridyldithio] propionyl)aminopentyl]aminobenzoate;

4′-amidinophenyl 4-N-[8-N-(3-[2-pyridyldithio] propionyl)amino-3,6-dioxaoctyl]aminobenzoate; or

4′-amidinophenyl 4-N-[2-N-(3-[2-pyridyldithio] propionyl)aminoethyl]aminobenzoate.

5. A process according to any preceding claim wherein the fibrinolytic enzyme is a plasminogen activator.

6. A process according to claim 5 wherein the fibrinolytic enzyme is tissue plasminogen activator or high molecular weight urokinase.

7. A process according to any preceding claim wherein the specific immunoglobulin is rabbit anti-[human erythrocyte membrane glycoprotein] immunoglobulin G or mouse monoclonal anti-(human fibrin) immunoglobulin G.

8. A process according to claim 1 for preparing 4-N-[2-N′-(3-[4′-butyrimino-(N″-ε-lys rabbit anti-[human erythrocyte membrane glycoprotein] immunoglobulin G)]dithiopropionyl) aminoethyl] aminobenzoyl-O-(ser-356) human high molecular weight urokinase,

4-N-[2-N′-(3-[4′-butyrimino-(N″-ε-lys rabbit anti-[human erythrocyte membrane glycoprotein] immunoglobulin G)]dithiopropionyl)aminoethyl] aminobenzoyl-O-(ser-478) human tissue plasminogen activator,

4-N-[8-N′-(3-[4′-butyrimino-(N″-ε- lys rabbit anti-[human erythrocyte membrane glycoprotein] immunoglobulin G)]dithiopropionyl)amino-3,6-dioxaoctyl] aminobenzoyl-O-(ser-478) human tissue plasminogen activator,

4-N-[2-N′-(3-[4′-butyrimino-(N″-ε-lys rabbit anti-[human erythrocyte membrane glycoprotein] immunoglobulin G)]dithiopropionyl)aminobutyl] aminobenzoyl- O-(ser-478) human tissue plasminogen activator,

4-N-[2-N′-(3-[4′-Butyrimino-(N″-ε-lys rabbit anti-[human erythrocyte membrane glycoprotein] immunoglobulin G)]dithiopropionyl)amino butyl]aminobenzoyl- O-(ser-478) human tissue plasminogen activator,

4-N-[2-N′-(3-[4′-butyrimino-(N″-ε-lys rabbit anti-[human erythrocyte membrane glycoprotein] immunoglobulin G)]dithiopropionyl)aminohexyl] aminobenzoyl-O-(ser-478) human tissue plasminogen activator, or

4-N-[2-N′-(3-[4′-butyrimino-(N″-ε-lys rabbit anti-[human erythrocyte membrane glycoprotein] immunoglobulin G)]dithiopropionyl)aminopropyl]aminobenzoyl-O-(ser-478) human tissue plasminogen activator.

9. A process according to claim 1 wherein the linking agent is of formula (II):

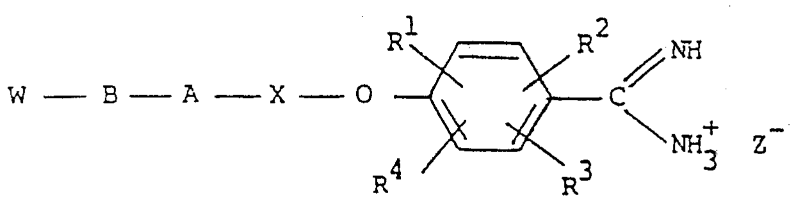

(II)

10. A process according to claim 9 wherein the linking agent of formula II is reacted with the immunoglobulin in a non-nucleophilic buffer at a temperature of 0°-30°C and a pH of 7-8 and the linking agent is reacted with the fibrinolytic enzyme in aqueous media at a pH which is not detrimental to the components of the reaction for example pH 4-8 at ambient or lower temperature.

11. A process for preparing a pharmaceutical composition comprising 0.1 to 99% of a derivative according to claim 1 in combination with a pharmaceutically acceptable carrier which process comprises admixing said derivative and carrier.

12. A method of treatment and/or prophylaxis of thrombotic disorders, which comprises administering to the mammal in need of such treatment and/or prophylaxis an effective non-toxic amount of a derivative according to claim 1.

13. The use of a derivative of claim 1, for the preparation of a medicament for the treatment and/or prophylaxis of thrombotic disorders.

14. A process for preparing a compound of formula (III)

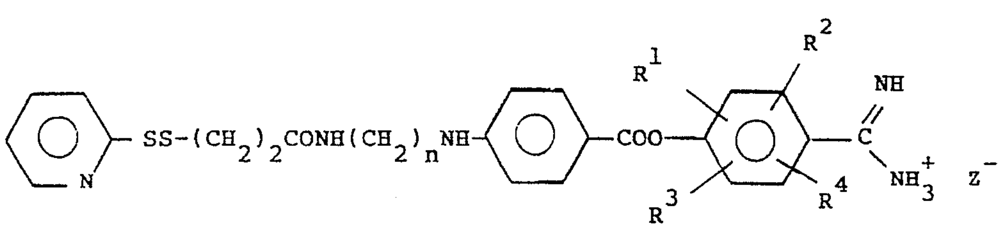

(III)

wherein n is an integer of 3 to 8, Z is a counter ion and each of $R^1$ to $R^4$ represents hydrogen or an electron withdrawing moiety which increases the reactivity of an amidinophenyl ester, which process comprises esterifying an acid of formula (IV):

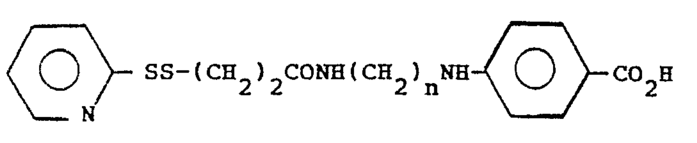

(IV)

wherein n is as defined in formula (III), with an alcohol of formula (V):

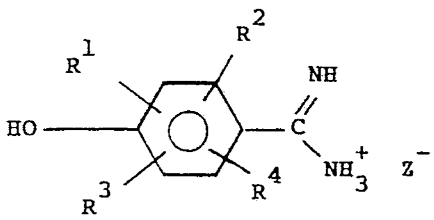

(V)

wherein $R^1$, $R^2$, $R^3$, $R^4$ and Z are as defined in formula (III).

15. A process according to claim 13 for preparing 4′-amidinophenyl 4-N-[4-N-(3-[2-pyridyldithio] propionyl)aminobutyl]aminobenzoate,

4′-amidinophenyl 4-N-[6-N-(3-[2-pyridyldithio] propionyl)aminohexyl]aminobenzoate,

4′-amidinophenyl 4-N-(3-N-[3-(2-pyridyldithio) propionyl]aminopropyl)aminobenzoate or

4′-amidinophenyl 4′-N-[5-N-(3-[2-pyridyldithio] propionyl)aminopentyl]aminobenzoate, or a salt thereof.

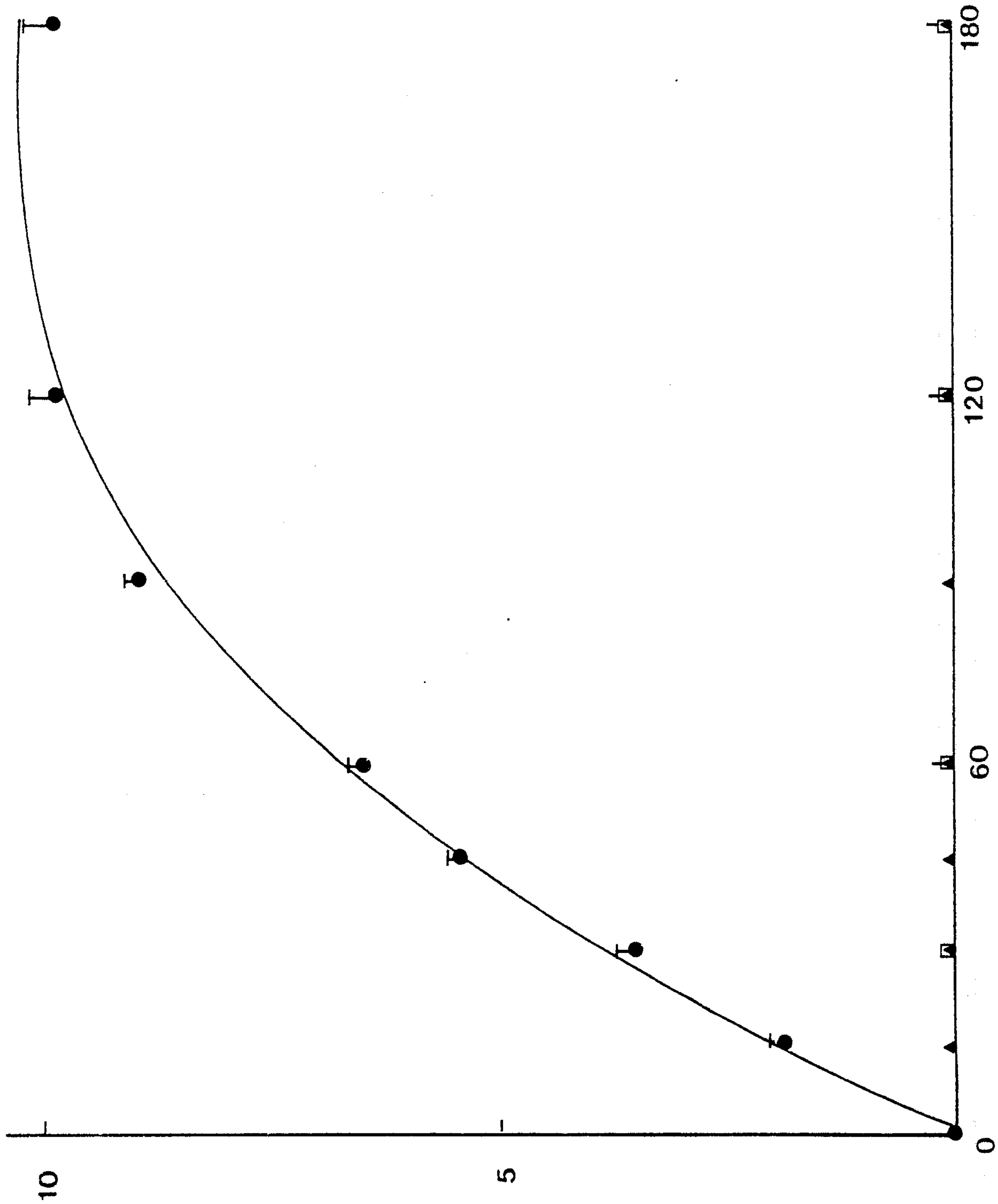


Fig.1

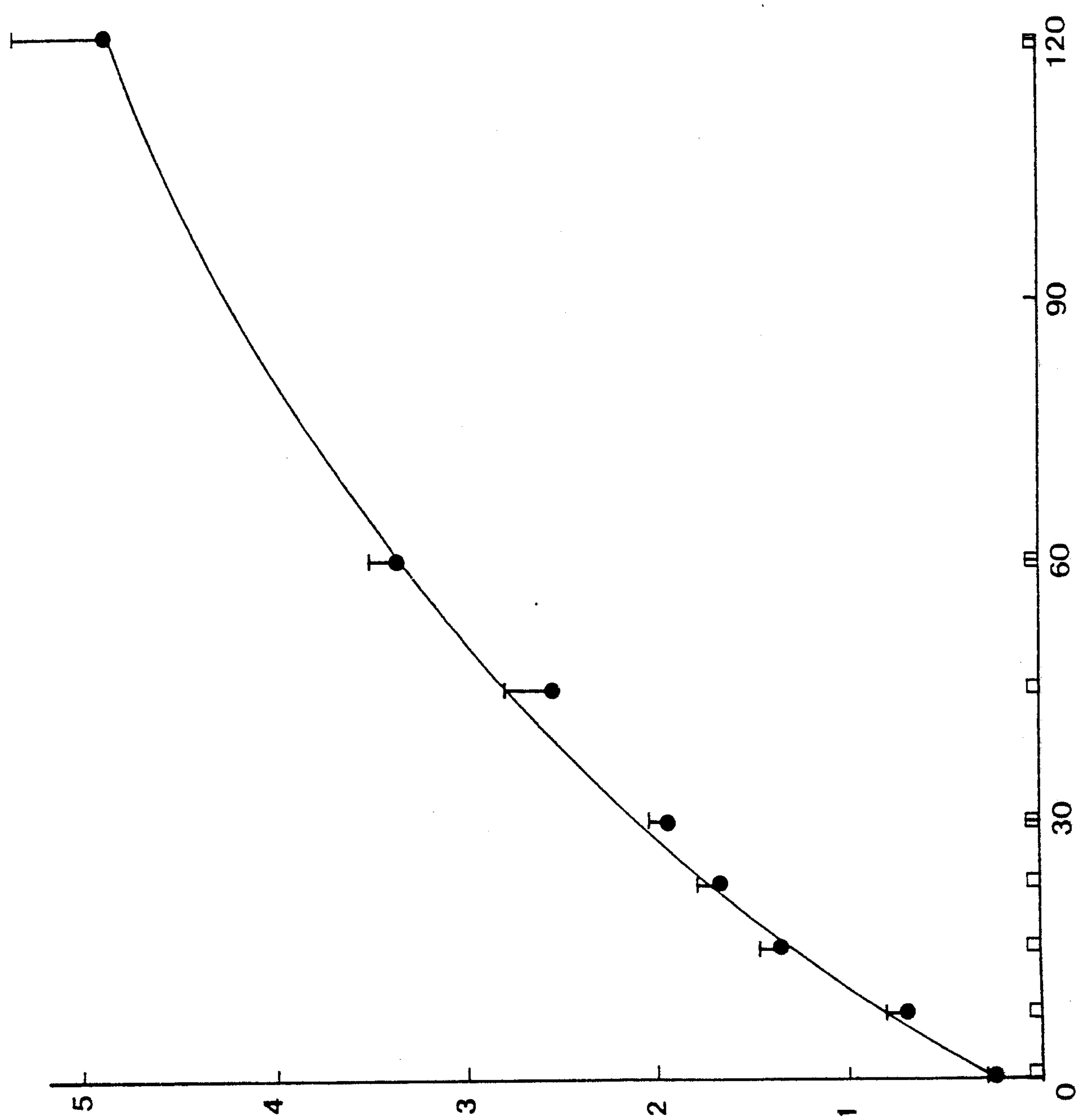


Fig.2